(19) Europäisches Patentamt
European Patent Office
Office européen des brevets

(11) Publication number: 0 001 516
A1

(12) EUROPEAN PATENT APPLICATION

(21) Application number: 78300473.2

(22) Date of filing: 09.10.78

(51) Int. Cl.[2]: C 07 D 498/04, A 61 K 31/42
// C07C69/52 ,(C07D498/04, 263/00, 205/00),(A61K31/43, 31/42),(A61K31/545, 31/42)

(30) Priority: 10.10.77 GB 42103/77

(43) Date of publication of application: 18.04.79
Bulletin 79/8

(84) Designated Contracting States: BE CH DE FR GB NL SE

(71) Applicant: GLAXO GROUP LIMITED, Clarges House 6-12 Clarges Street, London W1Y 8DH (GB)

(72) Inventor: Newall, Christopher Earle, 33 Elm Grove Road, Ealing London W.5 (GB)
Inventor: Tonge, Alan Paul, 130 Gunnersbury Lane, Acton London W.3 (GB)

(74) Representative: Holmes, Michael John et al, Frank B. Dehn & Co. Imperial House 15-19 Kingsway, London WC2B 6UZ (GB)

(54) **Beta-Lactam compounds, processes for their preparation, pharmaceutical compositions containing them and intermediates of use in their preparation.**

(57) β-Lactam compounds having the formula

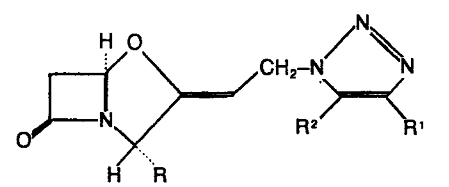

are described in which R is a carboxyl or esterified carboxyl group and $R^1$ and $R^2$, which may be the same or different, may each represent a hydrogen atom; a substituted or unsubstituted alkyl, aralkyl or aryl group; or a cyano, formyl, nitro, carboxyl, esterified carboxyl, etherified thiol or a sulphone derivative thereof, acyl, etherified hydroxyl, carbamoyl, substituted carbamoyl, sulphamoyl, substitued sulphamoyl, hydrazinocarbonyl or protected hydrazinocarbonyl group together with, in the case where R is a carboxyl group and/or $R^1$ and/or $R^2$ is a carboxyl group, salts thereof.

Processes for preparing the compounds and pharmaceutical compositions containing them are also disclosed and intermediates of use in their preparation are described. The compounds are of use as antibiotics or as β-lactamase inhibitors.

EP 0 001 516 A1

"β-Lactam compounds, processes for their preparation, pharmaceutical compositions containing them and intermediates of use in their preparation."

This invention relates to novel antibiotics, to a process for their production, to pharmaceutical compositions containing them and to intermediates of use in their preparation.

In our German OLS 2 604 697 we have described the isolation, from fermentations of Streptomyces clavuligerus, of clavulanic acid and salts thereof in pure form.

The compounds in this specification are named in general with reference to "clavam"; the name given to the parent heterocycle of formula (A)

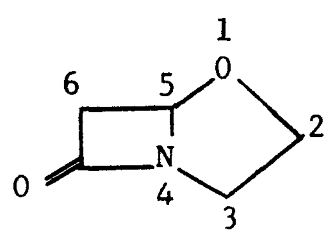

by analogy with the term "cepham" used in the naming of cephalosporin compounds in J. Amer. Chem. Soc., 1962, 84, 3400. Thus clavulanic acid is named (3R, 5R, Z)-2-(2-hydroxyethylidene)clavam-3-carboxylic acid.

The present invention relates to analogues of clavulanic acid and its salts and esters which carry an N- attached triazole group in place of the hydroxy group thereof. These are of use, as detailed below, as antibiotics or as β-lactamase inhibitors or they may be useful as intermediates in the preparation of further active compounds.

Accordingly, we provide compounds of the formula (I)

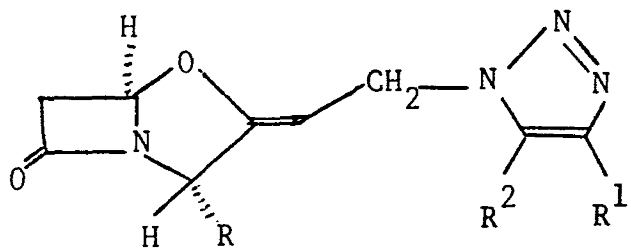

wherein R represents a carboxyl or esterified carboxyl group; $R^1$ and $R^2$, which may be the same or different, may each represent a hydrogen atom; a substituted or unsubstituted alkyl, aralkyl or aryl group; or a cyano, formyl,nitro,carboxyl, esterified carboxyl, etherified thiol or a sulphone derivative thereof, acyl, etherified hydroxyl, carbamoyl, substitued carbamoyl,sulphamoyl, substituted sulphamoyl, hydrazinocarbonyl or protected hydrazinocarbonyl group together with, in the case where R is a carboxyl group and/or $R^2$ is a carboxyl group, salts thereof.

When $R^1$ and/or $R^2$ are esterified carboxyl, acyl, etherified hydroxyl, or etherified thiol groups or sulphone derivatives of such thioether groups, they may be represented by the formulae $-CO_2R^4$, $-COR^4$, $-OR^4$, $-SR^4$ and $-SOR^4$ respectively where $R^4$ is a substituted or unsubstituted alkyl, aralkyl, aryl or carbon -attached heterocyclic group .

When $R^1$ and/or $R^2$ are carbamoyl or substituted carbamoyl, sulphamoyl or substituted sulphamoyl, or hydrazinocarbonyl or protected hydrazinocarbonyl groups they may be represented by the formulae $-CONR^5R^6$, $-SO_2NR^5R^6$ and $-CONHNHR^7$, where $R^5$ and $R^6$, which may be the same or different, may each represent a hydrogen atom, or a substituted or unsubstituted alkyl, aralkyl or aryl group or $R^5$ and $R^6$, together with the nitrogen atom to which they are attached, represent a 5-7 membered heterocyclic ring which may contain a further oxygen, sulphur or nitrogen atom, and $R^7$ may represent a hydrogen atom or a protecting group such as a benzyloxycarbonyl group.

When any of $R^1$,$R^2$,$R^4$,$R^5$ or $R^6$ is an alkyl group it will preferably have from 1-8 carbon atoms, for example methyl, ethyl, propyl or isopropyl, butyl, sec-butyl or tert-butyl. Such groups may if desired be substituted e.g. by a hydroxyl group or by one or more e.g. 1-3 halogen atoms such as fluorine, chlorine or bromine atoms.

When any of $R^1$,$R^2$,$R^4$,$R^5$ or $R^6$ is an aryl group it may have up to 12 carbon atoms, and may be substituted or unsubstituted and will preferably be monocyclic e.g. phenyl. Suitable substituents include nitro, halo and alkoxy, e.g. methoxy.

When any of $R^1$,$R^2$,$R^4$,$R^5$ or $R^6$ is an aralkyl group it may have up to 20 carbon atoms, but will preferably have up to six carbon atoms in the alkyl portion and will desirably be carbocyclic and, more preferably, monocyclic e.g. benzyl. The aralkyl group may be substituted or unsubstituted. Suitable substituents include a halogen atom or a nitro or alkoxy, e.g. methoxy, group.

When $R^4$ is a carbon-attached heterocyclic group it may contain 5-7 ring members and one or more (e.g. up to four) hetero- atoms such as oxygen, nitrogen or sulphur atoms. The heterocyclic group may be saturated or unsaturated, e.g. containing up to 3 double bonds in the ring.

Preferred groups $R^4$ include methyl, ethyl, propyl, isopropyl, butyl, phenyl, benzyl, trifluoromethyl, chloromethyl, 4-nitrobenzyl and 4-methoxybenzyl groups.

$R^5$ and $R^6$, which may be the same or different, preferably each represent a hydrogen atom or a methyl group, or the group $-NR^5R^6$ preferably represents a morpholino or piperidino group.

$R^7$ preferably represents a hydrogen atom.

It is preferred for one of $R^1$ and $R^2$ to be a hydrogen atom and compounds in which $R^2$ is a hydrogen atom are more preferred.

Compounds in which $R^2$ is a hydrogen atom and $R^1$ is a carbamoyl, carboxyl, acetyl, cyano, methoxycarbonyl, ethoxycarbonyl or N-methylcarbamoyl group are particularly preferred.

Preferred compounds are (3R,5R,Z)-2-[2-(4-carbamoyl -1,2,3-triazol-1-yl)ethylidene]-clavam-3-carboxylic acid and salts thereof, (3R,5R,Z)-2-[2-(4-acetyl-1,2,3-triazol-1-yl)ethylidene]-clavam-3-carboxylic acid and salts thereof, and (3R,5R,Z)-2-[2-(4-cyano-1,2,3 triazol-1-yl)ethylidene]-clavam-3-carboxylic acid and salts thereof.

The esters according to the invention may in general be represented as compounds of formula (I) in which R is a group $COOR^3$ where $R^3$ represents an organic group which is conveniently derived from an alcohol

(aliphatic or araliphatic), a phenol, a silanol, or a stannanol. Such an alcohol, phenol, silanol, or stannanol used to esterify the carboxyl group preferably contains not more than 24 carbon atoms.

Thus, the group $R^3$ may represent a straight or branched unsubstituted or substituted alkyl or alkenyl group, preferably having from 1-8 carbon atoms, for example a methyl, ethyl, propyl or isopropyl, butyl, sec-butyl, tert-butyl or allyl group, optional substituents being for example, alkoxy e.g. methoxy; halogen i.e. fluorine, chlorine, bromine or iodine; cyano; acyloxy, e.g. alkanoyloxy, such as acetoxy or pivaloyloxy or alkoxycarbonyloxy e.g. ethoxycarbonyloxy; acyl e.g. p-bromobenzoyl; and alkoxycarbonyl e.g. ethoxycarbonyl;

an aralkyl group having up to 20 carbon atoms especially an arylmethyl group e.g. a benzyl or substituted benzyl group, suitable substituents being either halo e.g. chloro; nitro, e.g. o- or p-nitro; cyano; alkoxy e.g. p-methoxy or alkyl e.g. p-methyl groups; a diphenylmethyl or triphenylmethyl group or a fur-2-ylmethyl, thien-2-ylmethyl or pyrid-4-ylmethyl group, the heterocyclic groups of which may also be substituted e.g. by a $C_{1-4}$ alkyl group, preferably methyl;

an aryl group having up to 12 carbon atoms e.g. a phenyl or substituted phenyl group, suitable substituents being either halo e.g. chloro; nitro, e.g. o- or p-nitro; cyano; alkoxy e.g. p-methoxy or alkyl e.g. p-methyl groups;

a cycloalkyl group containing not more than 12 carbon atoms e.g. adamantyl, cyclohexyl or cyclopentyl;

a heterocyclic group containing not more than 12 carbon atoms, the hetero atom being, for example, oxygen as in the tetrahydropyranyl or phthalidyl group;

a stannyl group having up to 24 carbon atoms for example a stannyl group carrying three substituents which may be the same or different selected from alkyl, alkenyl, aryl, aralkyl, cycloalkyl, alkoxy, aryloxy or aralkoxy groups. Such groups will include methyl, ethyl, propyl, n-butyl, phenyl and benzyl groups;

or a silyl group having up to 24 carbon atoms which may carry three groups which may be the same or different selected from alkyl, alkenyl, cycloalkyl, aralkyl and aryl groups. Such groups will preferably be $C_{1-4}$ alkyl e.g. methyl, ethyl, n-propyl, iso-propyl or t-butyl groups.

The salts of the invention may be salts formed with inorganic bases such as alkali metal salts, e.g. sodium, potassium and lithium salts; alkaline earth metal salts, e.g. calcium and magnesium salts and ammonium salts; or salts with organic bases, for example amine salts.

In general, the acids, salts and metabolically labile esters of the compounds of the invention are preferred forms for use in medicine. However, the other esters are also useful and, for example, where the ester group is readily cleaved e.g. by hydrolysis or hydrogenolysis, without significant degradation of the rest of the molecule, the esters are especially useful as carboxyl-protected derivatives of the parent acids. Those esters which are readily cleaved and are primarily of use in this connection include arylmethyl esters, especially the benzyl, p-nitrobenzyl, benzhydryl and trityl esters as well as the stannyl, e.g. tri-n-butylstannyl, and silyl, e.g. trimethylsilyl, esters. As

indicated above, the ester grouping may be metabolically labile, i.e. it may be converted into the carboxylic acid during metabolic processes, e.g. in the blood or liver. Metabolically labile esters include substituted alkyl esters carrying an oxygen substituent on the α-carbon atom, for example, the acyloxymethyl esters, e.g. acetoxymethyl and pivaloyloxymethyl esters, and the α-alkoxycarbonyloxyalkyl esters such as 1-ethoxycarbonyloxyethylesters, and phthalidyl esters.

The compounds of formula (I) and their salts for which we have demonstrated antibacterial activity have been active against a range of gram-negative and gram-positive microorganisms, for example against strains of Staphylococcus aureus, Escherichia coli, Salmonella typhimurium, Shigella sonnei, Enterobacter cloacae Klebsiella aerogenes, Proteus mirabilis, Proteus morganii, Serratia marcescens, Providencia species, Citrobacter koseri and Haemophilus influenzae.

In general, the compounds of formula (I) and their salts are stable to the action of β-lactamases produced by gram-positive organisms, for example those produced by Staphylococcus aureus, and to the β-lactamases produced by gram-negative organisms.

Compounds of the invention also possess the ability to inhibit β-lactamase enzymes produced by gram-positive organisms, for example those produced by strains of Staphylococcus aureus and also the enzymes from gram-negative bacteria produced by organisms such as strains of Proteus mirabilis, Escherichia coli, Proteus morganii, Klebsiella aerogenes, Salmonella typhimurium, Shigella

*sonnei*, *Haemophilus influenzae*, *Proteus vulgaris*, *Proteus rettgeri*, and *Citrobacter freundii*.

Compounds of the invention have the ability to protect β-lactamase susceptible β-lactam antibiotics from β-lactamase hydrolysis.

Certain compounds of the invention, including compounds of formula (I) in which R represents a carboxyl group in salt form, $R^2$ is a hydrogen atom and $R^1$ is an acetyl, cyano, or carbamoyl group, are also absorbed when administered orally as shown by studies in mice.

The new antibiotic acids and their physiologically acceptable salts and metabolically labile esters are of interest for use in conjunction with β-lactam antibiotics which show susceptibility to β-lactamases from both gram-positive and gram-negative organisms.

In general, active compounds of the invention may be used in combination with broad spectrum β-lactam antibiotics which are normally administered by either the oral or parenteral route. Use of the new antibiotic acids and their salts is preferred.

Examples of orally absorbed broad spectrum β-lactam antibiotics include cephalexin, cephaloglycin, ampicillin and amoxycillin and their orally absorbed esters, e.g. the acyloxymethyl and phthalidyl esters, and the orally absorbed esters of carbenicillin, ticarcillin and mecillinam e.g. the indanyl, phenyl and pivaloyloxymethyl esters. Broad spectrum β-lactam antibiotics which are not orally absorbed include carbenicillin, ticarcillin, mecillinam, cephalothin,

cephaloridine, cefazolin, cephacetrile and cephapirin. Examples of narrow spectrum β-lactam antibiotics are penicillin G and penicillin V.

The above antibiotics may also be in the form of their metabolically labile esters, pharmaceutically acceptable salts and/or solvents thereof.

Combinations of the active compounds of the invention with for example ampicillin and amoxycillin show synergistic activity against β-lactamase producing organisms including strains of for example Staphylococcus aureus, Escherichia coli, Klebsiella aerogenes, Proteus mirabilis, Salmonella typhimurium, Shigella sonnei, Proteus morganii and Proteus vulgaris.

According to a further feature of the invention, we provide pharmaceutical compositions (including veterinary compositions) containing at least one of the acids, physiologically acceptable salts or metabolically labile esters according to the invention. In view of the protective action described above the compositions can advantageously contain one or more,and more generally one,further β-lactam antibiotic. The compositions will normally also contain a pharmaceutical (including veterinary) carrier or excipient.

The compositions of the invention include those in a form adapted for oral or parenteral use.
The compositions may, for example, take the form of powders, tablets, capsules, lozenges, solutions and syrups suitable for oral administration, and may include, for example, starch, lactose, talc, magnesium stearate,

gelatin, distilled water and suspending, dispersing, emulsifying, flavouring or colouring agents.

The compounds may further be formulated in rectal compositions such as suppositories or retention enemas.

The compounds of the invention may be formulated for parenteral administration. The compounds may thus be formulated in ampoules for reconstitution before use.

In general, the weight ratio of the compound of the invention to a β-lactam antibiotic to be protected will be in the range 10:1 to 1:10, more preferably 5:1 to 1:5 especially 2:1 to 1:2.

The active compounds of the invention will generally be administered at a total daily dosage level of 50 mg to 20 g, preferably from 100 mg to 10 g, which may be in divided doses given from 1-4 times per day. Where the composition contains a further β-lactam antibiotic, the total quantity of β-lactam antibiotic will desirably be from 100 mg to 20 g, which may be given in divided doses from 1-4 times a day. In general, the total daily dosage of β-lactam antibiotic when the active compound is used alone or in combination with a further β-lactam antibiotic will advantageously be from 250 mg to 5 g. Dosage units will in general contain 12.5 mg to 5 g, preferably 50 mg to 1 g of active compound according to the invention when used alone and 25 mg to 5 g, preferably 100 mg to 1 g, of total β-lactam antibiotic where a further antibiotic is present.

Active compounds of the invention may be of use, either alone or in combination with a further β-lactam

antibiotic in treating a variety of diseases in humans and animals, caused by pathogenic bacteria, such as respiratory tract or urinary tract infections.

According to a further aspect of the invention, we provide a process for the preparation of a compound of formula (I) wherein a compound of formula (II)

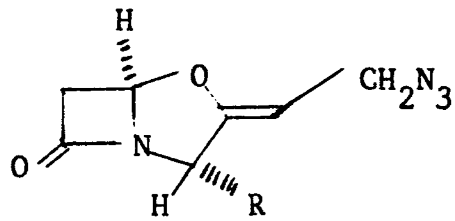

(II)

wherein R is as defined above is reacted with an acetylene

$R^1 - C \equiv C - R^2$

wherein $R^1$ and $R^2$ are as defined above,

followed, where the inital product is an ester and an acid or salt is desired, by deesterification, and by subsequent salt formation.

In this reaction R is preferably an esterified carboxyl group.

Reaction will generally be conducted in an inert organic solvent. Suitable solvents include ethers e.g. tetrahydrofuran, hydrocarbons e.g. benzene, halogenated hydrocarbons e.g. dichloromethane, amides e.g. dimethylformamide, or esters e.g. ethyl acetate.

A temperature of from 10° to 50° will normally be employed though higher temperatures may be needed with the less reactive acetylenes.

When an unsymmetrical acetylene reagent is used, ie. when $R^1$ and $R^2$ are different, a mixture of isomers may be obtained, although in general one isomer will predominate. It should be understood that this invention extends

to the individual isomers as well as mixtures containing them.

Azido compounds of formula (II) whence the compounds of the invention amy be prepared can be obtained as described in Belgian Patent No. 855,375.

In the above reactions, where the compound of the invention formed is an ester and the corrsponding acid or salt is required, the compound may be subjected to deesterification. For this purpose readily cleavable esters, for example, arylmethyl esters which may be cleaved by reduction, e.g. by hydrogenolysis, are preferred. Cleavage of an arylmethyl ester, e.g. a p-nitrobenzyl ester, may be effected by hydrogenolysis for example using a metal catalyst, e.g. a noble metal such as platinum, palladium or rhodium. The catalyst may be supported e,g. on charcoal or kieselguhr.

A p-nitrobenzyl group may also be removed by reduction of the nitro group (e.g. using a dissolving metal reducing agent such as zinc in acetic acid, or zinc in aqueous tetrahydrofuran or acetone controlled, for example, in the pH range 3-6, preferaby 4.0 - 5.5 by the addition of aqueous hydrochloric acid; aluminium amalgam in a moist ether, e.g. tetrahydrofuran; or iron and ammonium chloride in an aqueous ether e.g. aqueous tetrahydrofuran) followed by hydrolysis either under reduction conditions or by subsequent treatment with acid.

Alternatively, a p-nitrobenzyl ester may be cleaved under alkaline conditions using, for example, sodium sulphide in a solvent such as aqueous tetrahydrofuran,

dimethylformamide or acetone. A stannyl or silyl ester can be cleaved by very mild solvolysis, e.g. by reaction with water, alcohols, phenols or carboxylic acids, e.g. acetic acid.

The esters of formula (I) may be prepared from acids of formula (I) or a reactive derivative thereof by reaction with an alcohol, phenol, silanol or stannanol or a reactive derivative thereof to form the desired ester. Reaction will desirably be effected under mild conditions in order to prevent rupture of the bicyclic nucleus. The use of neutral or mild acidic or basic conditions, therefore, at temperatures between -70° and + 35°C is preferred.

The alkyl, alkoxyalkyl and aralkyl esters may be prepared by reaction of the acid of formula (I) with the appropriate diazoalkane or diazoaralkane e.g. diazomethane or diphenyldiazomethane. The reaction will generally be effected in an ether, ester or a halohydrocarbon solvent, e.g. diethyl ether, ethyl acetate or dichloromethane. In general, reduced temperatures are preferred, for example -15° to +15°C.

The esters derived from alcohols may be produced by reaction of a reactive derivative of the alcohol, for example, a halide such as the chloride, bromide or iodide, or a hydrocarbonsulphonyl derivative such as a methanesulphonyl or p-toluenesulphonyl ester, with a salt of the acid of formula (I) e.g. an alkali or alkaline earth metal salt such as a lithium, sodium, potassium, calcium or barium salt or an amine salt, such as a triethylammonium salt. This reaction is preferably carried out in a substituted sulphoxide or

amide solvent e.g. dimethyl sulphoxide, dimethylformamide or hexamethylphosphoramide.

Stannyl esters may conveniently be formed by reaction of the carboxylic acid of formula (I) or a salt thereof with reactive tetravalent tin moieties. Trialkyl tin oxides are preferred for the synthesis of tin compounds in view of their availability and low toxicity.

Silyl esters of the acid of formula (I) may be formed by reaction with a reactive tetravalent silicon moiety. Trialkylsilyl halides and mono- or bis-silylated acetamides are preferred as silylating agents. Proton acceptors e.g. weak bases such as pyridine may often be used with advantage when hydrogen halides are formed during such esterification.

In the formation of salts of the invention, an acid initially formed in solution in an appropriate organic solvent, may be reacted with an appropriate base, preferably under conditions favouring precipitation of the salt. In the formation of alkali metal salts, e.g. sodium or potassium salts, an alkanoate is a preferred base, e.g. a 2-ethylhexanoate.

The invention will now be more particularly described in the following Preparations and Examples which should not be construed as limiting the invention. Throughout the Examples all temperatures are in °C.

The spectral data obtained on compounds described in the Examples which follow was consistent with the structures assigned. Where n.m.r. data is presented, selected values only are given.

Preparation 1

4-Nitrobenzyl propiolate

A solution of propiolic acid (1.25 ml) and 4-nitrobenzyl alcohol (3.0 g) in benzene (100 ml) was refluxed in the presence of a few crystals of *p*-toluenesulphonic acid. Water which formed during the reaction was removed using a Dean-Stark attachment. After 7 days the mixture was passed down a column of silica gel (100 g), using ethyl acetate:petroleum ether (b.p. 60-80°) (2:3) eluent. Those fractions containing the product were combined and evaporated to dryness to give the title ester, m.p.43-44.5° (ether), τ ($CDCl_3$) 1.73 and 2.40 (aromatic protons), 4.60 (s, benzyl protons) and 6.94 (s, acetylene proton).

Example 1

4-Nitrobenzyl (3R,5R,Z)-2-[2-(4,5-diethoxycarbonyl-1,2,3-triazol-1-yl)-ethylidene]-clavam-3-carboxylate

A solution of diethyl acetylenedicarboxylate (0.25 g) and 4-nitrobenzyl (3R,5R,Z)-2-(2-azidoethylidene)-clavam-3-carboxylate (0.54 g) in tetrahydrofuran ( 3 ml) was allowed to stand at room temperature for 3 days. The mixture was chromatographed on preparative silica gel plates, using ethyl acetate:petroleum ether (b.p. 60-80°) (1:1) eluent, yielding the title compound (0.38 g), m.p. 104° (ethyl acetate:petroleum ether), $_{max}$($CHBr_3$) 1804 (β-lactam), 1750 and 1730 $cm^{-1}$ (ester).

Example 2

Sodium (3R,5R,Z)-2-[2-(4,5-diethoxycarbonyl-1,2,3-triazol-1-yl)-ethylidene]-clavam-3-carboxylate

Palladium on carbon (10%, 0.45 g) was added to a solution of 4-nitrobenzyl (3R,5R,Z)-2-[2-(4,5-diethoxycarbonyl-1,2,3-triazol-1-yl)ethylidene]clavam-3-carboxylate (0.59 g) in ethyl acetate (50 ml) and the mixture shaken on a hydrogenator until uptake of hydrogen ceased. The mixture was filtered through kieselguhr and a solution of sodium 2-ethylhexanoate (0.116 g) in ethyl acetate (10 ml) added to the filtrate. The filtrate was evaporated to a small volume and the concentrate diluted with ether (50 ml). The precipitated solid was filtered off and dried in a vacuum desiccator, yielding the title compound (0.33 g), $[\alpha]_D$ + 32° (c 0.88, $H_2O$), $\nu_{max}$ (Nujol) 1792 (β-lactam), 1730 $cm^{-1}$ ($-CO_2R$).

Example 3

4-Nitrobenzyl (3R,5R,Z)-2-[2-(4- and 5-ethoxycarbonyl-1,2,3-triazol-1-yl)-ethylidene]-clavam-3-carboxylate

A solution of ethyl propiolate (0.75 ml) and 4-nitrobenzyl (3R,5R,Z)-2-(2-azidoethylidene)-clavam-3-carboxylate (2.0 g) in benzene (10 ml) was warmed on an oil bath at 40° for 3 days. The mixture was then chromatographed on preparative silica gel plates using ethyl acetate:petroleum ether (b.p.40-60°) (2:1) eluent to give the 5-isomer of the title compound (0.20 g), $\nu_{max}$ ($CHBr_3$) 1800 (β-lactam), 1752 and 1725 $cm^{-1}$. (ester), τ ($CDCl_3$) 1.87 (s, triazole proton), 5.63 (q, J = 7 Hz, $-OC\underline{H}_2CH_3$) and 8.62 (t, J = 7 Hz, $-OCH_2C\underline{H}_3$) and 0.90 g of the 4-isomer of the title compound, $\nu_{max}$ ($CHBr_3$) 1802 (β-lactam), 1750 and 1716 $cm^{-1}$, (ester), τ ($CDCl_3$) 1.95 (s, triazole proton), 5.59 (q, J = 7 Hz, $-OC\underline{H}_2CH_3$), and 8.61 (t, J = 7 Hz, $-OCH_2C\underline{H}_3$).

Example 4

Sodium (3R,5R,Z)-2-[2-(4-ethoxycarbonyl-1,2,3-triazol-1-yl)-ethylidene]-clavam-3-carboxylate

Palladium on carbon (10%, 0.80 g) was added to a solution of 4-nitrobenzyl (3R,5R,Z)-2-[2-(4-ethoxycarbonyl-1,2,3-triazol-1-yl)ethylidene]clavam-3-carboxylate (0.82 g) in ethyl acetate (50 ml) and the mixture shaken on a hydrogenator until uptake of hydrogen ceased. The mixture was then filtered through kieselguhr and a solution of sodium 2-ethylhexanoate (0.19 g) in ethyl acetate (2 ml) added to the filtrate. The deposited solid was filtered off and dried in a desiccator, yielding the title compound (0.33 g) $\lambda_{max}$ (0.1 N aqueous NaOH)

258 nm ($\varepsilon$ = 15,400), $\nu_{max}$ (Nujol) 1792 ($\beta$-lactam) and 1728 $cm^{-1}$ (ester).

Example 5

4-Nitrobenzyl (3R,5R,Z)-2-[2-(4-acetyl-1,2,3-triazol-1-yl)-ethylidene]-clavam-3-carboxylate

A solution of 3-butyn-2-one (0.80 ml) and 4-nitrobenzyl (3R,5R,Z)-2-(2-azidoethylidene)-clavam-3-carboxylate (2.5 g) in benzene (15 ml) was allowed to stand at 38° for 5 days. The mixture was decanted from the reaction vessel and allowed to stand at room temperature for 30 min. The material which crystallized from solution was filtered off, dried and recrystallized from benzene:ethyl acetate (1:1) to give the title compound (0.75 g) m.p. 140-142°, $\nu_{max}$($CHBr_3$) 1804 ($\beta$-lactam) and 1754 $cm^{-1}$ (ester).

Example 6

Sodium (3R,5R,Z)-2-[2-(4-acetyl-1,2,3-triazol-1-yl)-ethylidene]-clavam-3-carboxylate

Palladium on carbon (10%, 0.60 g) was added to a solution of 4-nitrobenzyl (3R,5R,Z)-2-[2-(4-acetyl-1,2,3-triazol-1-yl)-ethylidene]-clavam-3-carboxylate (0.60 g) in ethyl acetate (50 ml) and the mixture shaken on a hydrogenator until uptake of hydrogen ceased. The mixture was filtered through kieselguhr and a solution of sodium 2-ethylhexanoate (0.17 g) in ethyl acetate (5 ml) added to the filtrate. The solution was concentrated to ca, 10 ml in vacuo and the deposited solid filtered off and dried in a

desiccator, yielding title compound (0.18 g), $_{max}$ (pH 6 buffer) 225 nm ($\varepsilon$ = 12,100), $\nu_{max}$ (Nujol) 1790 $cm^{-1}$ ($\beta$-lactam).

Example 7

4-Nitrobenzyl (3R,5R,Z)-2-{2-[4-[4-nitrobenzyloxycarbonyl)-1,2,3-triazol-1-yl]-ethylidene}-clavam-3-carboxylate

A solution of 4-nitrobenzyl propiolate (1.5 g) and 4-nitrobenzyl (3R,5R,Z)-2-(2-azidoethylidene)-clavam-3-carboxylate (2.7 g) in benzene (15 ml) was warmed on an oil bath at 45° for 3 days. The reaction mixture was then passed down a column of silica gel (150 g) and those fractions containing the product combined and evaporated to yield the title compound (1.0 g) as a foam, $\nu_{max}$ ($CHBr_3$) 1806 ($\beta$-lactam), 1750 and 1728 $cm^{-1}$ (ester), $\tau$ ($CDCl_3$) 1.86 (s, triazole proton), 4.20 (d, J = 2 Hz, C-5 H), and 4.78 (s, C-3 H).

Example 8

Disodium (3R,5R,Z)-2-[2-(4-carboxy-1,2,3-triazol-1-yl)-ethylidene]-clavam-3-carboxylate

Palladium on carbon (10% 1.8 g) was added to a solution of 4-nitrobenzyl (3R,5R,Z)-2-{2-[4-(4-nitrobenzyloxycarbonyl)-1,2,3-triazol-1-yl]-ethylidene-}clavam-3-carboxylate (0.90 g) in ethyl acetate (75 ml) and the mixture shaken on a hydrogenator until uptake of hydrogen ceased. The mixture was filtered through kieselguhr, the filtrate discarded and the filter cake

washed with ethanol (3 x 25 ml). The ethanolic washings were combined and added to a solution of sodium 2-ethylhexanoate (0.25 g) in ethanol (5 ml). The solution was concentrated to _ca._ 10 ml _in vacuo_ and the gelatinous precipitate filtered off and dried in a desiccator, yielding the title compound (0.14 g), $\lambda_{max}$ (0.1 N aqueous NaOH) 257.5 nm (ε = 14,800), $\nu_{max}$ (Nujol) 1788cm$^{-1}$ (β-lactam).

## Example 9

**4-Nitrobenzyl (3R,5R,Z)-2-[2-(4-and 5-acetyl-1,2,3-triazol-1-yl)-ethylidene]-clavam-3-carboxylate**

A solution of 3-butyn-2-one (1.2 ml) and 4-nitrobenzyl (3R,5R,Z)-2-(2-azidoethylidene)-clavam-3-carboxylate (3.6 g) in benzene (25 ml) was allowed to stand at 45° for 3 days. The mixture was then cooled to room temperature and, after leaving for 30 min, the deposited solid filtered off and dried. The solid was recrystallized from ethyl acetate to give the 4-isomer of the title compound (1.2 g), m.p. 140-142°, $\nu_{max}$ ($CHBr_3$) 1804 (β-lactam), and 1754 cm$^{-1}$ (ester).

The mother liquors from the recrystallization were combined with those from the reaction mixture and allowed to stand overnight in a stoppered flask. A solid was deposited which was filtered off and recrystallized three times from ethyl acetate to give the 5-isomer of the title compound (0.1 g), m.p. 143-145°, $\nu_{max}$ ($CHBr_3$) 1798 (β-lactam), and 1750 cm$^{-1}$ (ester).

Example 10

Sodium (3R,5R,Z)-2-[2-(5-acetyl-1,2,3-triazol-1-yl)-ethylidene]-clavam-3-carboxylate

A solution of 4-nitrobenzyl (3R,5R,Z)-2-[2-(5-acetyl-1,2,3-triazol-1-yl)-ethylidene]-clavam-3-carboxylate (0.20 g) in 1,4-dioxan (5 ml) was added to a suspension of palladium on carbon (10%, 0.25 g) in ethyl acetate (5 ml) and the mixture shaken on a hydrogenator for 5 min. The mixture was filtered through kieselguhr and a solution of sodium 2-ethylhexanoate (50 mg) in ethyl acetate (2 ml) added to the filtrate. The filtrate was concentrated to ca. 3 ml in vacuo and the concentrate diluted with ether (10 ml). The deposited solid was filtered off and dried in a desiccator to yield the title compound (90 mg) $\nu_{max}$(nujol) 1784 $cm^{-1}$($\beta$-lactam), $\tau$ ($D_2O$) 1.57 (s, triazole proton), 4.22 (d, J = 2 Hz, C-5H), 4.9-5.05 (m, =CH- and C-3H), and 7.37 (s, $-COCH_3$).

Example 11

4-Nitrobenzyl (3R,5R,Z)-2-[2-(4-carbamoyl-1,2,3-triazol-1-yl)-ethylidene]-clavam-3-carboxylate

A solution of propiolamide (1.4 g) and 4-nitrobenzyl (3R,5R,Z)-2-(2-azidoethylidene)-clavam-3-carboxylate ( 7.0 g) in tetrahydrofuran (35 ml) was warmed at 38° for one week. The solid which crystallized from solution was filtered off and dried, yielding the title compound (1.75 g), $\nu_{max}$ (Nujol) 1794 ($\beta$-lactam) and 1750 $cm^{-1}$(ester), $\tau$ (DMSC-$d_6$) 1.48 (s, triazole proton), 4.13 (d, J=2Hz, C 5H) and 4.40 (s, C-3H).

Example 12

Sodium (3R,5R,Z)-2-[2-(4-carbamoyl-1,2,3-triazol-1-yl)-ethylidene]-clavam-3-carboxylate

A solution of 4-nitrobenzyl (3R,5R,Z)-2-[2-(4-carbamoyl-1,2,3-triazol-1-yl)-ethylidene]-clavam-3-carboxylate (0.75 g) in 1,4-dioxan:ethyl acetate (2:1, 30 ml; ester dissolved by warming) was added to a suspension of palladium on carbon (10%, 1.0 g) in 1,4-dioxan:ethyl acetate (2:1, 30 ml) and the mixture shaken on a hydrogenator until uptake of hydrogen ceased. The mixture was filtered through kieselguhr and a solution of sodium 2-ethylhexanoate (0.20 g) in ethyl acetate (5 ml) added to the filtrate. The precipitated solid was filtered off, washed with ethyl acetate and dried in a desiccator to yield the title compound (0.32 g), $\lambda_{max}$ (0.1N NaOH) 257 nm ($\varepsilon$ 15,100), $\nu_{max}$ (Nujol) 1782 $cm^{-1}$ ($\beta$-lactam).

Example 13

4-Nitrobenzyl (3R,5R,Z)-2-{2-[4-(N-methylcarbamoyl)-1,2,-3-triazol-1-yl]-ethylidene}-clavam-3-carboxylate

A solution of N-methylpropiolamide (1.0 g) and 4-nitrobenzyl (3R,5R,Z)-2-(2-azidoethylidene)-clavam-3-carboxylate (4.5 g) in ethyl acetate (30 ml) was warmed at 38$^o$ for one week, then at 50$^o$ for a further week. The deposited tan powder was filtered off to give the title compound (0.38 g) and the mother liquors were cooled overnight at -5$^o$ to yield a further 0.50 g of product, $\nu_{max}$ (Nujol) 1786 ($\beta$-lactam) and 1738 $cm^{-1}$ (ester), $\tau$ (DMSO-$d_6$) 1.48 (s, triazole proton), 1.58

(broad singlet, $-CON\underline{H}CH_3$), 4.13 (d, J=2Hz, C-5H), 4.41 (s, C-3H), and 7.20 (d, $-CONHC\underline{H}_3$).

Example 14

Sodium (3R,5R,Z)-2-{2-[4-(N-methylcarbamoyl)-1,2,3-triazol-1-yl]-ethylidene}-clavam-3-carboxylate

A solution of 4-nitrobenzyl (3R,5R,Z)-2-{2-[4-(N-methylcarbamoyl)-1,2,3-triazol-1-yl]-ethylidene}-clavam-3-carboxylate (0.77 g) in 1,4-dioxan (20 ml) was added to a suspension of palladium on carbon (10%, 0.80 g) in ethyl acetate (20 ml) and the mixture shaken on a hydrogenator until uptake of hydrogen ceased. The mixture was then filtered through kieselguhr and a solution of sodium 2-ethylhexanoate (0.20 g) in ethyl acetate (5 ml) added to the filtrate. The precipitated solid was filtered off and dried in a desiccator, giving the title compound (0.35 g), $\lambda_{max}$ (0.1N NaOH) 256 nm ($\varepsilon$ 15,500), $\nu_{max}$ (Nujol) 1782 $cm^{-1}$ ($\beta$-lactam).

Example 15

4-Nitrobenzyl (3R,5R,Z)-2-[2-(4-formyl-1,2,3-triazol-1-yl)ethylidene]-clavam-3-carboxylate

A solution of propiolaldehyde (1.0 ml) and 4-nitrobenzyl (3R,5R,Z)-2-(2-azidoethylidene)-clavam-3-carboxylate (4.3 g) in benzene (20 ml) was allowed to stand at 38$^{o}$ for 4 days. The mixture was chromatographed on a column of silica gel (200 g) and those fractions containing the product combined and evaporated to a foam. The foam was recrystallised from a small volume of ethyl acetate (cooled to -10$^{o}$) to give 0.73 g of the title compound, m.p. 123-127$^{o}$, $\nu_{max}$ ($CHBr_3$) 1802

0001516

(β-lactam), 1752 (ester).

Example 16

4-Nitrobenzyl (3R,5R,Z)-2-[2-(4-cyano-1,2,3-triazol-1-yl)ethylidene]clavam-3-carboxylate

A solution of cyanoacetylene (0.52 g) and 4-nitrobenzyl (3R,5R,Z)-2-(2-azidoethylidene)clavam-3-carboxylate (2.5 g) in benzene (20 ml) was heated at 35-40$^{o}$ for 5 days. The mixture was concentrated and the residue passed down a column of silica gel, using ethyl acetate:petroleum ether (1:1) eluent, to give the title ester (1.23 g), $\nu_{max}$ ($CHBr_3$) 1804 (β-lactam), 1752 $cm^{-1}$ (ester), τ ($CDCl_3$) 1.91 (s, triazole proton), 4.18 (d, J 2Hz, C-5H), 4.76 (s, C-3H) and 4.8-5.0 (m, =CH- and $-CH_2N<$).

Example 17

Sodium (3R,5R,Z)-2-[2-(4-cyano-1,2,3-triazol-1-yl)-ethylidene]clavam-3-carboxylate

A solution of 4-nitrobenzyl (3R,5R,Z)-2-[2-(4-cyano-1,2,3-triazol-1-yl)ethylidene]clavam-3-carboxylate (0.60 g) in ethyl acetate (25 ml) was shaken on a hydrogenator over palladium on carbon (10%, 0.60 g) for 20 min. After this time, a further quantity of palladium on carbon (10%, 0.6 g) was added and hydrogenolysis continued for another 15 min. The mixture was then filtered through Kieselguhr and the filter pad washed with ethanol. The filtrate and washings were combined and treated with a solution of sodium 2-ethylhexanoate (0.17 g) in ethyl acetate (10 ml). The mixture was concentrated to ca 5 ml

in vacuo, then diluted with ether (15 ml). The precipitated solid was filtered off, yielding the title compound (0.22 g), $\nu_{max}$ (Nujol) 2240 (-CN), 1788 $cm^{-1}$ (β-lactam), τ ($D_2O$) 1.42 (s, triazole proton), 4.22 (d, J 2Hz, C-5H), 4.8-4.9 (m, =CH- and $-CH_2Ar$) and 4.99 (s, C-3H).

Example 18

4-Nitrobenzyl (3R,5R,Z)-2-[2-(4-methoxycarbonyl-1,2,3-triazol-1-yl)ethylidene]clavam-3-carboxylate

A solution of methyl propiolate (1.68 g) and 4-nitrobenzyl (3R,5R,Z)-2-(2-azidoethylidene)clavam-3-carboxylate (3.59 g) in ethyl acetate (50 ml) was warmed at 45$^{o}$ for 3 days, then allowed to stand at room temperature for a further 3 days. The reaction mixture was concentrated and chromatographed on a column of silica gel, using ethyl acetate:petroleum ether (bp. 40-60$^{o}$) (3:2) eluent to yield the title compound (1.7 g), $\nu_{max}$ ($CHBr_3$) 1800 (β-lactam), 1750 and 1720 $cm^{-1}$ (esters), τ ($CDCl_3$) 1.96 (s, triazole proton), 4.22 (d, J 3Hz, C-5H), 4.8-5.0 (m, =CH-, C-3H and $-CH_2N<$) and 6.08 (s, $-OCH_3$).

Example 19

Sodium (3R,5R,Z)-2-[2-(4-methoxycarbonyl-1,2,3-triazol-1-yl)ethylidene]clavam-3-carboxylate

A solution of 4 nitrobenzyl (3R,5R,Z)-2-[2-(4-methoxycarbonyl-1,2,3-triazol-1-yl)ethylidene]clavam-3-carboxylate (0.77 g) in ethyl acetate (25 ml) was hydrogenated over palladium on carbon (10%, 0.80 g) until uptake of hydrogen ceased. The mixture was

filtered through Kieselguhr and the filter cake washed with ethanol (4 x 10 ml). The combined filtrate and washings were treated with a solution of sodium 2-ethylhexanoate (0.20 g) in ethyl acetate (5 ml) and the mixture concentrated to ca 5 ml. The concentrate was diluted with ether (10 ml) and the precipitated solid filtered off to yield the title compound (0.31 g) $\nu_{max}$ (Nujol) 1785 (β-lactam), 1725 $cm^{-1}$ (ester), τ ($D_2O$) 1.52 (s, triazole proton), 4.23 (d, J 3Hz, C-5H), 4.90 (=CH- and $-CH_2N=$) and 5.00 (s, C-3H).

Example 20

Acetoxymethyl (3R,5R,Z)-2-[2-(4-acetyl-1,2,3-triazol-1-yl)ethylidene]clavam-3-carboxylate

Chloromethyl acetate (0.22 ml) was added to a solution of sodium iodide (0.72 g) in acetone (5 ml) and the mixture stirred at room temperature for 2 hr. The mixture was evaporated to near dryness and partitioned between petroleum ether (bp. 40-60°) and water. The organic phase was dried ($Na_2SO_4$) and added to a suspension of sodium (3R,5R,Z)-2-[2-(4-acetyl-1,2,3-triazol-1-yl)ethylidene]clavam-3-carboxylate (0.45 g) in dimethylformamide (5 ml). After stirring for 20 min, the mixture was partitioned between brine (20 ml) and ethyl acetate (20 ml). The organic phase was washed with water (2 x 20 ml), dried ($Na_2SO_4$) and concentrated in vacuo. The residue was chromatographed on a column of silica gel, using ethyl acetate: petroleum ether (2:1) eluent, to give the title

compound (0.04 g), $\nu_{max}$ ($CHBr_3$) 1800 (β-lactam), 1762 (ester) and 1685 $cm^{-1}$ (acetyl), τ ($CDCl_3$) 1.96 (s, triazole proton), 4.24 (m, C-5H and $-OCH_2O-$), 4.8-5.0 (m, C-3H, = CH- and $-CH_2N=$), and 7.92 (s, $-OCOCH_3$).

Example 21

4-Nitrobenzyl (3R,5R,Z)-2-[2-(1,2,3-triazol-1-yl)-ethylidene]clavam-3-carboxylate

Acetylene was slowly passed through a solution of 4-nitrobenzyl (3R,5R,Z)-2-(2-azidoethylidene)clavam-3-carboxylate (0.50 g) in dioxan (10 ml) at 70° for five hrs. The reaction mixture was concentrated and the residue passed down a column of silica gel, eluting initially with ethyl acetate:petroleum ether (bp 40-60°) (2:1) and finally with ethyl acetate, to give the title compound (10 mg), $\nu_{max}$ ($CHBr_3$) 1800 (β-lactam), 1750 $cm^{-1}$ (ester), τ ($CDCl_3$) 2.28 and 2.48 (singlets, triazole protons), 4.20 (d, C-5H), 4.8-5.0 (m, = CH-, C-3H and $-CH_2N<$).

Example 22

Sodium (3R,5R,Z)-2-[2-(1,2,3-triazol-1-yl)ethylidene]-clavam-3-carboxylate

A solution of 4-nitrobenzyl (3R,5R,Z)-2-[2-(1,2,3-triazol-1-yl)ethylidene]clavam-3-carboxylate (0.24 g) in ethyl acetate (25 ml) was hydrogenated at atmospheric pressure over 10% palladium on carbon (0.35 g) until uptake of hydrogen ceased. The mixture was filtered through kieselguhr and the filtrate treated with a solution of sodium 2-ethylhexanoate (0.08 g) in ethyl acetate (3 ml). The resulting mixture was concentrated to a small volume and the deposited solid filtered off yielding the title compound (0.09 g) $\nu_{max}$ (Nujol) 1785 (β-lactam) and 1620 $cm^{-1}$ (ester), τ ($D_2O$) 2.00 and 2.18 (triazole protons) and 4.18 (d,J 3Hz, C-5 H).

Example 23

4-Nitrobenzyl (3R5R,Z)-2-[2-(4-acetyl-5-phenyl-1,2,3-triazol-1-yl)ethylidene]clavam-3-carboxylate

A solution of 4-nitrobenzyl (3R,5R,Z)-2-(2-azidoethylidene)clavam-3-carboxylate (1.0 g) and 4-phenyl-3-butyn-2-one (0.45 ml) in ethyl acetate (5 ml) was heated at 70° for 6 days. The mixture was concentrated and chromatographed on silica gel plates, using ethyl acetate: petroleum ether (1:1) eluent, to give the title compound (0.18 g), $\nu_{max}$ ($CHBr_3$) 1800 (β-lactam), 1750 (ester) and 1680 $cm^{-1}$ (acetyl), τ ($CDCl_3$) 2.5-2.75 (m, phenyl protens), 4.45 (d, J 3Hz, C-5 H) 4.95 (s, C-3 H) and 7.36 (s, $-COCH_3$).

0001516

Example 24

4-Nitrobenzyl(3R,5R,Z)-2-[2-(5-ethoxy-1,2,3-triazol-1-yl)ethylidene]clavam-3-carboxylate

Ethoxyacetylene (0.25 ml of a 60% solution in hexane) was added to a solution of 3R,5R,Z-2-(2-azidoethylidene)clavam-3-carboxylate (0.50 g) in dioxan (2 ml) and the mixture heated in a sealed flask at 80-85° for 18 hr. The mixture was then concentrated and passed down a column of silica gel, using ethyl acetate:petroleum ether (bp 40-60°) (2:1) eluent, to give the title compound (50 mg), $\nu_{max}$ ($CHBr_3$) 1800 (β-lactam) and 1732 $cm^{-1}$ (ester), τ ($CDCl_3$) 2.98 (s, triazole proton), 4.26 (d, J 3Hz. C-5H), 4.88 (s, C-3H), 5.90 (q, J 7Hz, $-OC\underline{H}_2CH_3$) and 8.60 (t, J 7Hz, $-OCH_2C\underline{H}_3$).

Examples A - C

In Examples A and B, sodium (3R,5R,Z)-2-[2-(4-cyano-1,2,3-triazol-1-yl)ethylidene]-clavam-3-carboxylate, sodium (3R,5R,Z)-2-[2-(4-carbamoyl-1,2,3-triazol-1-yl)-ethylidene]clavam-3-carboxylate and ampicillin trihydrate are used as densified granules containing 1% w/w magnesium stearate, which are prepared as follows:

Blend the antibiotic with 1% magnesium stearate and prepare tablet slugs by direct compression on a tablet machine. Break down the slugs through a series of screens (10, 12, 16 and 20 mesh) on a rotary granulator to produce free flowing densified granules with an apparent bulk density of about 0.7 g/ml (BSS method).

Example A

Formula per tablet

| | |
|---|---|
| Densified sodium (3R,5R,Z)-2-[2-(4-cyano-1,2,3-triazol-1-yl) ethylidene]clavam-3-carboxylate containing 1% magnesium stearate | 252.5 mg |
| Empicol LZ | 3.5 mg |
| Explotab | 7.0 mg |
| Avicel pH 101 to tablet core weight of | 350.0 mg |

Method of preparation

Blend the granules with the rest of the excipients and compress the blend on a tablet machine using normal or deep concave punches of appropriate diameter (9-12 mm).

Example B

Formula per capsule

| | |
|---|---|
| Densified sodium (3R,5R,Z)-2-[2-(4-carbamoyl-1,2,3-triazol-1-yl)ethylidene]-clavam-3-carboxylate containing 1% magnesium stearate | 252.5 mg |
| Densified ampicillin trihydrate granules containing 1% magnesium stearate equal to 250 mg ampicillin (approx.) | 300.0 mg |
| Explotab | 10.0 mg |
| Aerosil 200 | 5.0 mg |

Method of preparation

Blend the granules and excipients and fill the blend into size 0 hard gelatin capsules (lock fitting type) on an automatic capsule-filling machine.

Example C

Dry powder for injection

Blend sterile sodium (3R,5R,Z)-2-[2-(4-ethoxycarbonyl-1,2,3-triazol-1-yl)-ethylidene]clavam-3-carboxylate (1 part) intimately under aseptic conditions with sterile cephazolin sodium (1 part). Fill the sterile blend aseptically into glass vials under a blanket of sterile nitrogen such that each vial contains 500 mg of each antibiotic. Close the vials using rubber discs or plugs held in position by aluminium sealing rings, thereby preventing gaseous exchange or ingress of micro-organisms. Constitute the product by dissolving in Water for Injections shortly before administration. Other suitable sterile vehicles can be used in place of Water for Injections.

Empicol LZ is sodium lauryl sulphate available

from Marchon Ltd; Explotab is sodium starch glycolate available from Greeff Fine Chemicals Ltd., Croydon, Surrey, England; and Avicel pH 101 is microcrystalline cellulose available from FMC Corporation, U.S.A.

Biological testing of a number of compounds of the invention has provided the results shown in the following tables; the test compounds are identified by their Example Numbers:

RESULTS OF BIOLOGICAL TESTS OF CLAVULANIC ACID DERIVATIVES

1. Determination of Minimum Inhibitory Concentrations (MIC) - Table 1

Serial two-fold dilutions of freshly prepared test solution were made into Oxoid No. 1 Nutrient agar with or without added enrichment and poured into petri dishes. Plates were innoculated with a multipoint innoculator with innoculum containing approximately $10^5$ colony forming units of the organisms shown in Table 1, all of which are clinical isolates. The MIC in µg/ml was read after 18 hours incubation at 37°C as the lowest concentration which inhibited growth.

2. Synergy testing-Table 3

Method as for 1, incorporating amoxycillin in serial two-fold dilutions along with a sub-inhibitory, fixed concentration of the test clavulanic acid derivative, as shown in the Table. All of the organisms are clinical isolates.

3. β-Lactamase Inhibition - Table 2

The compounds have been tested for their inhibitory activity against partially purified β-lactamases from Escherichia coli (either TEM or RPI), Klebsiella aerogenes (K1) and Staphylococcus aureus (PCI). The substrate used was ampicillin sodium. The results are shown in Table 2.

Ampicillin sodium at a concentration of 500 μg/ml was reacted with the enzyme at 37°C and pH7. The enzyme concentration was such as to destroy all the ampicillin within 10 minutes. The reaction was followed spectrophotometrically by observing the loss of absorption at 238 nm. Concentrations of the compound were added to inhibit the reaction, and the $I_{50}$ value represents μg/ml of compound halving the initial velocity of hydrolysis of ampicillin.

4. Toxicity

The compounds have generally been found to be of low toxicity. Thus, for example, the compounds of Examples 4, 6, 14, 17 and 19 when administered intraperitoneally to mice gave $LD_{50}$ values of greater than 500 mg/kg.

TABLE 1

Antibiotic Activity of various Clavulanic Acid Derivatives

| Organism | | MIC µg/ml (agar dilution) of compound | | | |
|---|---|---|---|---|---|
| Example No: | | 17 | 4 | 6 | 8 |
| Staph. aureus | 835E | <0.1 | <0.1 | <0.1 | 8 |
| Micrococcus SP | 1810E | - | <0.1 | <0.1 | 8 |
| E. coli | 851E | 2 | 4 | 4 | 8 |
| E. cloacae | 1051E | 4 | 16 | 8 | 16 |
| E. cloacae | 1321E | 4 | 16 | 8 | 8 |
| K. aerogenes | 1522E | 8 | 8 | 8 | 16 |
| S. marcescens | 1324E | 31 | 125 | 62 | 31 |
| H. influenzae | 1184E | 16 | 62 | 62 | 31 |

| Organism | | MIC µg/ml (agar dilution) of compound | | | |
|---|---|---|---|---|---|
| Example No: | | 12 | 14 | 19 | 21+ |
| Staph. aureus | 853E | 0.5 | 1 | 0.5 | 2 |
| Micrococcus SP | 1810E | 0.2 | 1 | - | - |
| E. coli | 851E | 4 | 8 | 4 | 4 |
| E. cloacae | 1051E | 4 | 16 | 16 | 8 |
| E. cloace | 1321E | 4 | 16 | 16 | 8 |
| K. aerogenes | 1522E | 8 | 16 | 16 | 8 |
| S. marcescens | 1324E | 8 | 31 | 62 | 8 |
| H. influenzae | 1184E | 31 | 31 | 62 | 16 |

+ As the sodium salt

Table 2

Inhibition by clavulanic acid derivatives of the destruction of Ampicillin by partially-purified β-lactamases

| β-lactamase | μg/ml of compound giving 50% inhibition of hydrolysis of ampicillin | | | | |
|---|---|---|---|---|---|
| Example No: | 17 | 2 | 4 | 6 | 8 |
| PCI | 0.7 | 2.1 | 0.135 | 0.18 | 2.9 |
| TEM | 0.1 | 0.3 | - | - | 0.11 |
| RPI | - | - | 0.074 | 0.18 | - |
| KI | 0.39 | 0.8 | 0.35 | 0.155 | 0.22 |

| β-lactamase | μg/ml of compound giving 50% inhibition of hydrolysis of ampicillin | | | |
|---|---|---|---|---|
| Example No: | 12 | 10 | 14 | 21+ |
| PCI | 0.36 | 0.7 | 0.67 | 1.3 |
| TEM | 0.125 | 0.58 | - | 0.215 |
| RPI | - | - | 0.36 | - |
| KI | 0.34 | 0.62 | 0.7 | 0.55 |

+ As the sodium salt

Table 3

MIC of amoxycillin alone or in the presence of 4 or 1μg/ml of compound of Example indicated

| Organism | MIC of amoxycillin μg/ml | | | | |
|---|---|---|---|---|---|
| | | Example 17 | | Example 4 | |
| | Alone | 4μg/ml | 1μg/ml | 4μg/ml | 1μg/ml |
| Staph. aureus 853E | 2 | * | * | * | * |
| E. coli (TEM+) 1193E | > 250 | * | 31 | * | > 250 |
| Pr. rettgeri 1356E | > 250 | 2 | 31 | 4 | 125 |
| K. aerogenes 1963E | 62 | 0.5 | 2 | * | 2 |
| | | Example 12 | | Example 14 | |
| | Alone | 4μg/ml | 1μg/ml | 4μg/ml | 1μg/ml |
| Staph. aureus 853E | 2 | * | * | * | < 0.1 |
| E. coli (TEM+) 1193E | > 250 | 1 | 62 | 4 | 62 |
| Pr. rettgeri 1356E | > 250 | 1 | 8 | 1 | 8 |
| K. aerogenes 1963E | 62 | < 0.1 | 1 | 0.2 | 8 |

* compound alone was inhibition

\+ As the sodium salt

Table 3

(Cont.)

MIC of amoxycillin alone or in the presence of 4 or 1 µg/ml of compound of Example indicated

| Organism | MIC of amoxycillin µg/ml | | | | |
|---|---|---|---|---|---|
| | | Example 6 | | Example 8 | |
| | Alone | 4µg/ml | 1µg/ml | 4µg/ml | 1µg/ml |
| Staph. aureus 853E | 2 | * | * | * | < 0.1 |
| E. coli (TEM+) 1193E | > 250 | * | 31 | 4 | 125 |
| Pr. rettgeri 1356E | > 250 | * | 16 | < 0.1 | 2 |
| K. aerogenes 1963E | 62 | * | 0.5 | < 0.1 | 0.5 |
| | | Example 19 | | Example 21 | |
| | Alone | 4µg/ml | 1µg/ml | 4µg/ml | 1µg/ml |
| Staph. aureus 853E | 2 | * | * | * | < 0.1 |
| E. coli (TEM+) 1193E | > 250 | 8 | 250 | < 0.1 | 16 |
| Pr. rettgeri 1356E | > 250 | 1 | 16 | 0.5 | 8 |
| K. aerogenes 1963E | 62 | 0.5 | 1 | < 0.1 | 1 |

* compound alone was inhibition

\+ As the sodium salt

0001516

Patent Claims

1. Compounds of the formula (I)

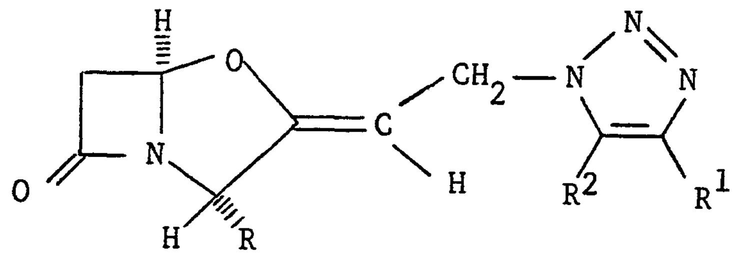

(I)

wherein R represents a carboxyl or esterfied carboxyl group and $R^1$ and $R^2$, which may be the same or different, each represent a hydrogen atom; a substituted or unsubstituted alkyl, aralkyl or aryl group; or a cyano, formyl, nitro, carboxyl, esterified carboxyl, etherified thiol or a sulphone derivative thereof, acyl, etherified hydroxyl, carbamoyl, substitued carbamoyl, sulphamoyl, substituted sulphamoyl, hydrazinocarbonyl or protected hydrazinocarbonyl group together with, when R, and/or $R^1$ and/or $R^2$ is a carboxyl group, salts thereof.

2. Compounds as claimed in claim 1 wherein R represents group - $COOR^3$ wherein $R^3$ represents hydrogen; a straight or branched substituted or unsubstituted alkyl or alkenyl group having from 1 - 8 carbon atoms; an aralkyl group having up to 20 carbon atoms; an aryl group having up to 12 carbon atoms; or a cycloalkyl group having up to 12 carbon atoms; a heterocyclic group having up to 12 carbon atoms, or a silyl or stannyl group having up to 24 carbon atoms, and $R^1$ and/or $R^2$ represents a group of formula $-CO_2R^4$, $-CO.R^4$, $-OR^4$, $-SR^4$, $-SO.R^4$, $-CONR^5R^6$, $-SO_2NR^5R^6$ or $-CONHNHR^7$ wherein $R^4$ is a substituted or unsubstituted alkyl, aralkyl, aryl or carbon-attached heterocyclic group; $R^5$ and $R^6$, which may be the same or different, each represent hydrogen, substituted or unsubstituted alkyl,aralkyl or aryl or $R^5$ and $R^6$, together

with the nitrogen atom to which they are attached, represent a 5 - 7 membered heterocyclic ring which optionally contains a further oxygen, sulphur or nitrogen atom, and $R^7$ represents hydrogen or a protecting group.

3. Compounds as claimed in claim 2 wherein $-COOR^3$ is a metabolically labile ester group.

4. Compounds as claimed in claim 2 wherein $R^3$ is a diphenylmethyl group, a benzyl group or a benzyl group substituted by an o- or p-nitro, p-methoxy or p-methyl group, a methyl group or an acetoxymethyl group.

5. Compounds as claimed in any of claims 1 - 5 in which one of $R^1$ and $R^2$ is a hydrogen atom.

6. Compounds as claimed in any of claims 1 - 6 in which $R^2$ is a hydrogen atom and $R^1$ is a carbamoyl, carboxyl, acetyl, cyano, methoxycarbonyl, ethoxycarbonyl or N-methylcarbamoyl group.

7. (3R, 5R,Z)-2-[2-(4-Carbamoyl -1,2,3-triazol-1-yl)-ethylidene]-clavam-3-carboxylic acid and salts thereof

8. (3R,5R,Z)-2-[2-(4-Acetyl-1,2,3-triazol-1-yl)-ethylidene]-clavam-3-carboxylic acid and salts thereof.

9. (3R,5R,Z)-2-[2-(4-Cyano-1,2,3-triazol-1-yl)ethylidene]-clavam-3-carboxylic acid and salts thereof.

10. The alkali metal, alkaline earth metal, ammonium and organic base salts of compounds as claimed in any of claims 1- 9.

11. A pharmaceutical composition (including a veterinary composition) comprising at least one acid, physiologically acceptable salt or metabolically labile ester of a compound of formula (I) as claimed in claim 1 in admixture with a pharmaceutical carrier or excipient and/or a further β-lactam antibiotic.

12. A process for the preparation of compounds of formula (I) as claimed in claim 1 wherein a compound of formula (II)

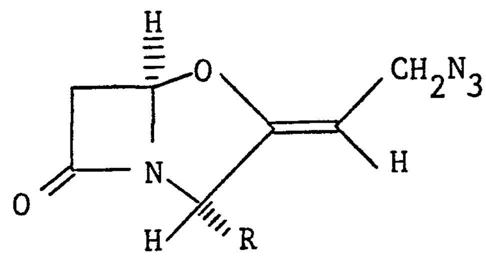

(II)

wherein R is as defined in claim 1 is reacted with an acetylene $R^1-C\equiv C-R^2$ wherein $R^1$ and $R^2$ are as defined in claim 1, followed, where the initial product is an ester and an acid or salt is required, by deesterification and by subsequent salt formation.

13. A process as claimed in claim 12 wherein R is an esterified carboxyl group.

0001516

European Patent Office

# EUROPEAN SEARCH REPORT

Application number

EP 78 30 0473

## DOCUMENTS CONSIDERED TO BE RELEVANT

| Category | Citation of document with indication, where appropriate, of relevant passages | Relevant to claim |
|---|---|---|
| A | GB - A - 1 211 694 (GLAXO)<br>* Page 3, lines 47-62; page 4 and pages 7-9 *<br>-- | 1,12 |
| A(P) | BE - A - 860 042 (GLAXO)<br>* Claims *<br>---- | 1,11 |

CLASSIFICATION OF THE APPLICATION (Int. Cl.²)

C 07 D 498/04
A 61 K 31/42//
C 07 C 69/52
(C 07 D 498/04
263/00
205/00)
(A 61 K 31/43
31/42)
(A 61 K 31/545
31/42)

TECHNICAL FIELDS SEARCHED (Int.Cl.²)

C 07 D 498/04
A 61 K 31/42
31/43
31/545

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 20-12-1978 | CHOULY |